# FASCICULE DE BREVET EUROPEEN

(11) **EP 3 551 282 B1**
(45) Date de publication et mention de la délivrance du brevet: **03.11.2021**
(21) Numéro de dépôt: 17808521.3
(22) Date de dépôt: 07.12.2017
(51) Int. Cl.: A61N 1/372, A61B 5/024, A61N 1/36

(54) **SYSTÈME IMPLANTABLE**
IMPLANTIERBARES SYSTEM
IMPLANTABLE SYSTEM

(30) Priorité: 07.12.2016 FR 1662059
(43) Date de publication de la demande: 16.10.2019
(73) Titulaire: Université Grenoble Alpes, 38400 Saint-Martin-d'Hères (FR); Centre Hospitalier Universitaire Grenoble Alpes, 38700 La Tronche (FR)
(72) Inventeur: CINQUIN, Philippe, 38330 Saint Nazaire les Eymes (FR); BOUCHER, François, Régis, Pierre, 38000 Grenoble (FR); DEFAYE, Pascal, 38000 Grenoble (FR); GUMERY, Pierre-Yves, 38000 Grenoble (FR); TUVIGNON, Patrick, 81000 Albi (FR)
(74) Mandataire: Ex Materia
(86) Numéro de dépôt international: PCT/EP2017/081877
(87) Numéro de publication internationale: WO 2018/104472

(56) Documents cités:
- WO-A2-02/089655
- WO-A2-2006/010025
- US-A1- 2005 090 873
- US-A1- 2006 111 753

## Description

La présente invention concerne un système implantable.

Un grand nombre de dispositifs implantables sont utilisés pour surveiller ou stimuler certains organes du corps humain. Par exemple, des dispositifs de stimulation cardiaques (ou « pacemakers ») sont implantés dans de nombreux patients. Ces dispositifs comportent en général une source d'énergie telle qu'une batterie, un ou plusieurs capteurs permettant de surveiller le comportement de l'organe surveillé et/ou un module de stimulation prévu pour exercer une action sur l'organe stimulé. Le document WO2006010025 relate l'art antérieur.

Cependant, il est nécessaire de recharger ou de remplacer régulièrement les batteries de tels dispositifs implantés. En particulier, dans de nombreux cas ce remplacement est effectué par une opération chirurgicale. Une telle procédure est relativement chère et contraignante pour le patient puisqu'elle a lieu dans un bloc opératoire d'un établissement hospitalier et qu'une anesthésie est nécessaire, ainsi qu'un séjour prolongé dans l'établissement hospitalier afin de surveiller les suites de l'opération. En outre, comme pour toute intervention chirurgicale, il existe des risques que le patient contracte une infection lors de l'opération.

Dans d'autres cas, des dispositifs implantés du type précité sont alimentés depuis l'extérieur par un module de stockage d'énergie qui est porté par le patient à l'extérieur de son corps. Par exemple, il arrive que des dispositifs d'alimentation transmettent de l'énergie par ondes ultrasonores au dispositif de stimulation, à travers la peau et la cage thoracique du patient. Cependant, les ondes ultrasonores traversent mal les os, et une grande précision dans le placement de la source d'ultra-sons est alors nécessaire, dans les cas où le dispositif implanté est situé dans la cage thoracique, afin d'assurer une bonne alimentation du dispositif implanté. En outre, un tel dispositif d'alimentation extérieur au corps du patient est disgracieux.

Il arrive également que des dispositifs implantables soient équipés de connecteurs filaires, permettant une connexion électrique ou un transfert de fluide entre le dispositif implantable et un dispositif extérieur. Par ce biais, un courant électrique d'alimentation ou des données mesurées par les capteurs du dispositif implanté sont échangés avec le dispositif extérieur. Là encore, ces connecteurs débouchant à travers la peau du patient sont disgracieux, et présentent nécessairement des risques sanitaires ainsi que des contraintes importantes pour le patient dans sa vie de tous les jours.

Il existe donc un besoin pour un système implantable qui soit moins contraignant pour le patient.

A cet effet, il est proposé un système implantable comprenant :
- un premier dispositif, appelé dispositif de centralisation, propre à être fixé dans une position de fixation à une paroi de l'estomac d'un patient, le dispositif de centralisation étant accueilli dans l'estomac lorsque le dispositif de centralisation est dans la position de fixation, et
- au moins un deuxième dispositif,
le système implantable étant caractérisé en ce que le dispositif de centralisation comprend un contrôleur, une alimentation électrique et un émetteur/récepteur propre à permettre la communication entre le dispositif de centralisation et le deuxième dispositif lorsque le deuxième dispositif est dans une position de fonctionnement, le deuxième dispositif étant situé en-dehors du corps du patient lorsque le deuxième dispositif est dans la position de fonctionnement.

Selon des modes de réalisation, le système implantable comprend l'une ou plusieurs des caractéristiques suivantes, prises isolément ou suivant toutes les combinaisons techniquement possibles :
- le dispositif de centralisation est configuré pour communiquer avec chaque deuxième dispositif par communication radiofréquence ;
- le dispositif de centralisation comporte un capteur propre à mesurer une valeur d'un paramètre physiologique du patient, l'émetteur/récepteur étant configuré pour transmettre à un deuxième dispositif les valeurs mesurées, le deuxième dispositif considéré étant propre à détecter un phénomène physiologique du patient à partir d'au moins une des valeurs mesurées ;
- le système implantable comprend deux deuxièmes dispositifs, et l'un des deuxièmes dispositifs comporte au moins un capteur propre à mesurer une valeur d'un paramètre physiologique du patient, l'émetteur/récepteur étant configuré pour recevoir du deuxième dispositif comportant un capteur les valeurs mesurées et pour transmettre les valeurs mesurées à l'autre deuxième dispositif, l'autre deuxième dispositif étant propre à détecter un phénomène physiologique du patient à partir d'au moins une des valeurs reçues de l'émetteur/récepteur ;
- le système implantable comporte une mémoire propre à stocker les valeurs mesurées pendant une durée supérieure ou égale à un jour ;
- le phénomène physiologique est une pathologie cardiaque ;
- la pathologie cardiaque est une insuffisance cardiaque ;
- le capteur est un émetteur/récepteur d'ultrasons ;
- le capteur est un accéléromètre propre à mesurer des valeurs d'une accélération du dispositif de centralisation et le deuxième dispositif est configuré pour calculer un paramètre physiologique du cœur du patient à partir des valeurs d'accélération mesurées ;
- le capteur est propre à mesurer une différence de potentiel électrique, une accélération, un bruit, une orientation, un pH ou une température ;
- le dispositif de centralisation comprend un cathéter propre à conduire un fluide corporel du patient au capteur, le capteur étant propre à mesurer un taux d'un marqueur biologique dans le fluide corporel ;
- le capteur comporte au moins une source lumineuse et au moins un détecteur de rayonnement lumineux, la source lumineuse étant configurée pour illuminer au moins une portion d'un organe du patient avec un rayonnement lumineux, le détecteur étant configuré pour mesurer une valeur d'un taux de réflexion du rayonnement lumineux, le contrôleur étant configuré pour calculer un taux d'oxygénation du sang circulant dans l'organe illuminé par la source lumineuse, à partir du taux de réflexion ;
- le système comprend une pluralité de capteurs, et le deuxième dispositif est propre à détecter le phénomène physiologique à partir de valeurs d'au moins deux capteurs ;
- le phénomène physiologique est choisi parmi l'ensemble formé de : un trouble du rythme cardiaque, une fibrillation auriculaire, une syncope, une insuffisance cardiaque, une apnée du sommeil, une broncho-pneumopathie chronique obstructive, un emphysème, une épilepsie, un trouble de la déglutition, un trouble de l'alimentation ;
- lorsque le dispositif de centralisation est dans la position de fixation, le dispositif de centralisation est dans la partie supérieure de l'estomac ;
- l'alimentation électrique comporte une réserve d'énergie électrique amovible et un connecteur propre à accueillir la réserve d'énergie électrique, la réserve d'énergie électrique étant propre à alimenter électriquement le contrôleur lorsque la réserve d'énergie électrique est connectée électriquement au connecteur dans une position de connexion et de préférence étant configurée pour être avalée par le patient et pour se déplacer spontanément jusqu'à la position de connexion depuis une position de déconnexion dans laquelle la réserve d'énergie électrique est accueillie dans l'estomac du patient et est déconnectée du connecteur ;
- l'alimentation électrique comporte un générateur d'énergie électrique propre à générer un courant électrique par réaction d'au moins une espèce chimique présente dans le corps du patient, notamment le glucose ;
- l'alimentation électrique comporte un générateur d'énergie électrique propre à générer un courant électrique par conversion d'énergie mécanique en énergie électrique.

Des caractéristiques et avantages de l'invention apparaîtront à la lecture de la description qui va suivre, donnée uniquement à titre d'exemple non limitatif et faite en référence aux dessins annexés, sur lesquels :
- la figure 1 est un schéma d'un exemple de système implantable comportant une alimentation électrique,
- la figure 2 est une représentation schématique du dispositif implantable de la figure 1, implanté dans le corps d'un patient, et
- la figure 3 est une représentation schématique de l'alimentation de la figure 1. Un premier exemple de système implantable 10 est représenté à la figure 1.

Le système implantable 10 comporte une ancre 15, un premier dispositif 20, appelé dispositif de centralisation, et au moins un deuxième dispositif 25.

Il est entendu par « système implantable » qu'au moins un élément parmi la liste formée de l'ancre 15 du premier dispositif 20 et du deuxième dispositif 25 est prévu pour être implanté dans le corps humain.

En particulier, il est entendu par « implantable » qu'au moins un élément parmi l'ancre 15, le dispositif de centralisation 20 et le deuxième dispositif 25 est prévu pour séjourner dans le corps d'un patient P pendant une durée strictement supérieure à une semaine, de préférence strictement supérieure à un mois, de préférence supérieure ou égale à un an.

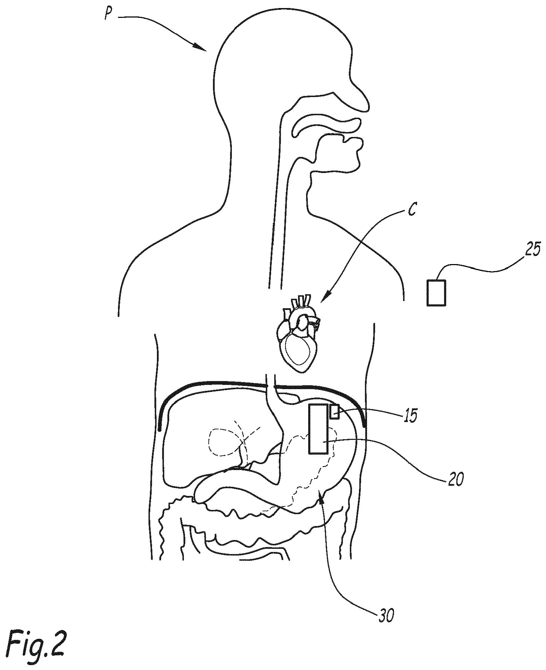

Le système implantable 10 a été représenté schématiquement sur la figure 2 lorsque le système implantable 10 est implanté dans le corps du patient P.

Le système implantable 10 est configuré pour détecter au moins un phénomène physiologique se produisant chez le patient P. Il est entendu par « phénomène physiologique » un phénomène concernant une fonction d'un organe du corps du patient P. Le phénomène physiologique est une pathologie cardiaque. Par exemple, le phénomène physiologique est un trouble du rythme cardiaque. Par exemple, le phénomène physiologique est une fibrillation auriculaire du cœur du patient P.

En variante, le phénomène physiologique est une syncope. Selon une autre variante, le phénomène physiologique est une bradycardie.

Selon une autre variante, le phénomène physiologique est une insuffisance cardiaque du cœur du patient P.

Selon l'exemple de la figure 2, l'ancre 15 et le dispositif de centralisation 20 sont chacun implanté dans le corps du patient P. Le deuxième dispositif 25 a été représenté dans une position de fonctionnement sur la figure 2.

L'ancre 15 est propre à être fixée dans une position prédéterminée dans l'estomac 30 du patient P.

Par exemple, l'ancre 15 est configurée pour être fixée dans la partie supérieure de l'estomac 30. En particulier, l'ancre 15 est configurée pour être fixée dans le fundus gastrique de l'estomac 30. Par exemple, l'ancre 15 est prévue pour être fixée le plus près possible de l'angle de Hiss dans le fundus gastrique.

En variante, l'ancre 15 est configurée pour être fixée dans la partie inférieure de l'estomac 30.

L'ancre 15 est configurée pour supporter le dispositif de centralisation 20, de préférence de manière amovible. En particulier, l'ancre 15 et le dispositif de centralisation 20 sont configurés pour être fixés l'un à l'autre, par un dispositif de fixation, et l'ancre 15 est configurée pour maintenir le dispositif de centralisation 20 dans une position de fixation lorsque l'ancre 15 est fixée dans l'estomac 30.

L'ancre 15 comporte une tête 35 et un premier connecteur 40.

La tête 35 est configurée pour ancrer l'ancre 15 dans la position prédéterminée. En particulier, la tête 35 est configurée pour ancrer l'ancre 15 à la paroi de l'estomac 30.

La tête 35 est, par exemple, un clip gastro-intestinal configuré pour enserrer entre deux branches de la tête 35 une portion de la paroi de l'estomac 30.

En variante, la tête 35 est propre à être suturée par un fil à la paroi de l'estomac 30.

Selon une autre variante, la tête 35 est propre à être enfouie à l'intérieur de la muqueuse gastrique après que celle-ci ait été disséquée.

Le premier connecteur 40 est configuré pour fixer le dispositif de centralisation 20 à la tête 35.

Le dispositif de centralisation 20 comporte au moins un capteur 117, un premier contrôleur 45, un deuxième connecteur 50, une alimentation électrique 55, un premier émetteur/récepteur 60 et un boîtier 65. Par exemple, le dispositif de centralisation 20 comporte deux capteurs 117.

Chaque capteur 117 est extérieur au premier contrôleur 45 mais est propre à communiquer avec le premier contrôleur 45.

Chaque capteur 117 est configuré pour mesurer des valeurs d'un paramètre physiologique du patient P. Le paramètre physiologique est, par exemple, un paramètre d'un organe C.

L'organe C est distinct de l'estomac du patient P. Par exemple, au moins un capteur 117 est propre à mesurer des valeurs d'un paramètre du cœur.

Par exemple, un capteur 117 est propre à mesurer une valeur d'une accélération du dispositif de centralisation 20, telle qu'une accélération causée par une contraction du cœur C.

En variante ou en complément, un capteur 117 est propre à mesurer une valeur d'une différence de potentiel électrique entre deux électrodes du capteur 117. La différence de potentiel électrique est, par exemple, mesurée entre deux points de la paroi stomacale, c'est-à-dire que les deux électrodes sont en contact avec la paroi stomacale. En variante, le capteur 117 ne comporte qu'une électrode, et est propre à mesurer la différence de potentiel électrique entre l'électrode et l'ancre 15. En variante, l'ancre 15 comporte deux électrodes et le capteur 117 est propre à mesurer une différence de potentiel entre les deux électrodes de l'ancre 15.

Le premier contrôleur 45 est une unité de traitement d'informations. Le premier contrôleur 45 comporte une première mémoire 75 et un premier processeur 80.

La première mémoire 75 est propre à stocker les valeurs mesurées par le capteur 117 pendant une période de stockage supérieure ou égale à une heure, de préférence supérieure ou égale à un jour, de préférence supérieure ou égale à une semaine.

Le premier processeur 80 est propre à manipuler et/ou transformer des données représentées comme des quantités électroniques ou physiques dans la première mémoire 75 en d'autres données similaires correspondant à des données physiques dans la première mémoire 75, dans des registres ou d'autres types de dispositifs d'affichage, de transmission ou de mémorisation.

Le premier processeur 80 est, en outre, configuré pour échanger des informations avec le premier émetteur/récepteur 60.

Le deuxième connecteur 50 est configuré pour coopérer avec le premier connecteur 40 pour maintenir le dispositif de centralisation 20 dans la position de fixation.

Par exemple, le deuxième connecteur 50 est configuré pour coopérer avec le premier connecteur 40 par encliquetage.

En variante, le deuxième connecteur 50 comporte un aimant configuré pour fixer le deuxième connecteur au premier connecteur. L'aimant est, par exemple, un électroaimant.

Selon une autre variante, le premier connecteur 40 est configuré pour être solidarisé au deuxième connecteur 50 par vissage. En variante, le premier connecteur 40 comporte une ou de préférence deux baïonnettes complémentaires d'orifices de fixation ménagés dans le deuxième connecteur 50.

De préférence, le deuxième connecteur 50 est prévu pour que le dispositif de centralisation 20 soit séparable de l'ancre 15. En particulier, le deuxième connecteur 50 est configuré pour que le dispositif de centralisation 20 soit séparable de l'ancre 15 lorsque l'ancre 15 est fixée dans l'estomac 30 du patient P.

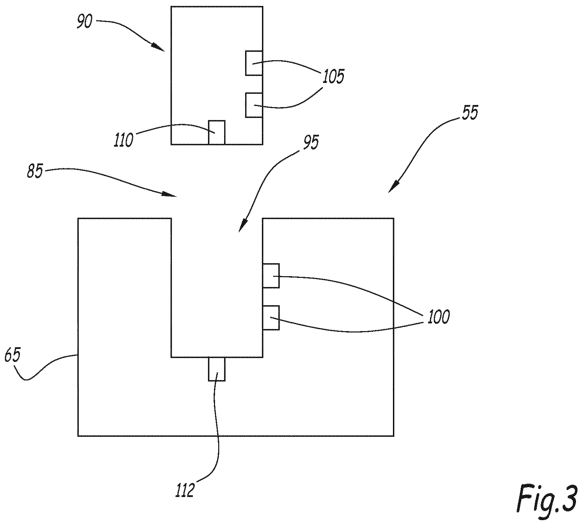

L'alimentation électrique 55 a été représentée sur la figure 3.

L'alimentation électrique 55 est configurée pour alimenter le premier contrôleur 45 avec un courant d'alimentation C.

L'alimentation électrique 55 comporte un troisième connecteur 85 et une première réserve d'énergie électrique 90.

Le troisième connecteur 85 est configuré pour recevoir de la première réserve d'énergie électrique 90 le courant d'alimentation C et pour alimenter le premier contrôleur 45 avec le premier courant d'alimentation C.

Le troisième connecteur 85 est configuré pour accueillir la première réserve d'énergie électrique 90. En particulier, le troisième connecteur 85 délimite une cavité 95 configurée pour accueillir au moins partiellement la première réserve d'énergie électrique 90 dans une position de connexion.

Selon l'exemple de la figure 3, la cavité 95 débouche sur l'extérieur du boîtier 65. En particulier, la cavité 95 est configurée pour permettre l'insertion de la première réserve d'énergie électrique 90, depuis l'extérieur du boîtier 65, dans la cavité 95.

Le troisième connecteur 85 comporte, en outre, deux premiers contacts électriques 100, configurés pour être connectés électriquement à la première réserve d'énergie électrique 90 lorsque la première réserve d'énergie électrique 90 est dans la position de connexion. En particulier, les deux premiers contacts électriques 100 débouchent à l'intérieur de la cavité 95.

La première réserve d'énergie électrique 90 est configurée pour stocker de l'énergie électrique. En particulier, la première réserve d'énergie électrique 90 est configurée pour être chargée en énergie électrique à l'extérieur du corps du patient P et pour se décharger lorsque la première réserve d'énergie électrique 90 est dans la position de connexion. Par exemple, la première réserve d'énergie électrique 90 comporte une batterie. En variante, la première réserve d'énergie électrique 90 comporte au moins un capaciteur ou une supercapacité.

La première réserve d'énergie électrique est configurée pour alimenter le premier contrôleur 45 avec le courant d'alimentation C lorsque la première réserve d'énergie électrique 90 est dans la position de connexion.

Selon l'exemple de la figure 3, la première réserve d'énergie électrique 90 comporte deux deuxièmes contacts électriques 105 complémentaires des premiers contacts électriques 100.

La première réserve d'énergie électrique 90 peut être prévue pour être avalée par le patient P.

Selon une variante, la première réserve d'énergie 90 est propre à être remplacée par endoscopie.

En particulier, la première réserve d'énergie électrique 90 présente un volume strictement inférieur à 6 millilitres (ml).

La première réserve d'énergie électrique 90 présente, en outre, trois dimensions mesurées chacune selon une direction respective, chaque direction étant perpendiculaire aux deux autres directions, et chaque dimension est strictement inférieure à 5 centimètres (cm).

La première réserve d'énergie électrique 90 est mobile entre la position de connexion et une position de déconnexion. Lorsque la première réserve d'énergie électrique 90 est dans la position de déconnexion, la première réserve d'énergie électrique 90 est accueillie dans l'estomac 30 du patient P mais n'est pas connectée électriquement au troisième connecteur 85. Par exemple, lorsque la première réserve d'énergie électrique 90 est dans la position de déconnexion, la première réserve d'énergie électrique est totalement extraite de la cavité 95.

La première réserve d'énergie électrique 90 est configurée pour se déplacer spontanément depuis la position de déconnexion vers la position de connexion. Par exemple, la première réserve d'énergie électrique 90 comporte des attracteurs 110.

De préférence, la première réserve d'énergie électrique 90 est configurée pour éjecter du troisième connecteur 85 une éventuelle autre première réserve d'énergie électrique 90 usagée. En d'autres termes, la première réserve d'énergie électrique 90 est configurée pour, si une première réserve d'énergie électrique 90 usagée est dans la position de connexion, provoquer la déconnexion de la première réserve d'énergie électrique 90 usagée et le déplacement de la première réserve d'énergie électrique usagée depuis la position de connexion jusqu'à la position de déconnexion.

Les attracteurs 110 sont configurés pour exercer sur la première réserve d'énergie électrique 90, lorsque la première réserve d'énergie électrique 90 est dans la position de déconnexion, une force tendant à déplacer la première réserve d'énergie électrique 90 depuis la position de déconnexion vers la position de connexion.

En outre, les attracteurs 110 sont configurés pour maintenir la première réserve d'énergie électrique 90 dans la position de connexion.

Les attracteurs 110 comportent, par exemple, un premier aimant propre à coopérer avec un deuxième aimant 112 du troisième connecteur 85. En variante, le premier aimant est propre à coopérer avec une portion ferromagnétique du troisième connecteur 85. Le premier aimant et le deuxième aimant 112 sont, par exemple, des électro-aimants.

Le premier émetteur/récepteur 60 est configuré pour échanger des informations avec le deuxième dispositif 25 lorsque le deuxième dispositif 25 est dans la position de fonctionnement. Le premier émetteur/récepteur 60 forme ainsi des moyens de communication avec le deuxième dispositif 25.

Le premier émetteur/récepteur 60 est, par exemple, un module de communication radiofréquence. Il est entendu par « module de communication radiofréquence » que le premier émetteur/récepteur 60 est configuré pour communiquer avec le deuxième dispositif 25 via un signal comportant au moins une onde électromagnétique radiofréquence. Les ondes électromagnétiques radiofréquences sont les ondes électromagnétiques présentant une fréquence comprise entre 3 kilohertz et 3 gigahertz.

Selon un mode de réalisation, le premier émetteur/récepteur 60 est propre à échanger des informations avec le deuxième dispositif 25 selon un protocole Bluetooth Low Energy. Le protocole Bluetooth Low Energy est un protocole basé sur un standard du « Bluetooth spécial interest group » et fonctionnant dans la gamme comprise entre 2400 mégahertz (MHz) et 2483.5 MHz.

En variante, des modes de transmission d'information dans les gammes 402 - 405 mégahertz (MHz) (Medical Implant Communication Service) ou 2360 - 2390 MHz (Medical Body Area Networks) peuvent être utilisés.

Le boîtier 65 est configuré pour isoler le premier contrôleur 45 de l'extérieur du boîtier 65. Par exemple, le boîtier 65 délimite une chambre recueillant au moins le premier contrôleur 45 et le premier émetteur/récepteur 60.

Le deuxième dispositif 25 n'est pas implanté à l'intérieur du corps du patient P lorsque le dispositif 25 est dans la position de fonctionnement. En particulier, le deuxième dispositif 25 est situé en-dehors du corps du patient P lorsque le deuxième dispositif 25 est dans la position de fonctionnement.

Par exemple, le deuxième dispositif 25 est installé dans le cabinet d'un médecin. En variante, le deuxième dispositif 25 est installé au domicile du patient P. En variante, le deuxième dispositif 25 est porté par le patient, par exemple le deuxième dispositif 25 est un téléphone mobile tel qu'un smartphone, une tablette, un appareil dédié ou encore un module intégré à un téléphone mobile ou à une tablette.

Le deuxième dispositif 25 est un dispositif d'affichage propre à transmettre des informations à un utilisateur U.

Le deuxième dispositif 25 est, en outre, configuré pour détecter le phénomène physiologique à partir des valeurs mesurées.

Le deuxième dispositif 25 comporte un deuxième émetteur/récepteur 120, un deuxième contrôleur 135 et une interface homme/machine 140.

Le deuxième émetteur/récepteur 120 est configuré pour échanger des informations avec le premier émetteur/récepteur 60.

Le deuxième contrôleur 135 est configuré pour détecter le phénomène physiologique à partir des valeurs mesurées par le capteur 117.

Le deuxième contrôleur 135 comporte une deuxième mémoire 145 et un deuxième processeur 150.

L'interface homme/machine comporte, par exemple, un écran et un haut-parleur.

Un procédé de surveillance de l'organe C est mis en œuvre par le système implantable 10.

Le procédé de surveillance comporte une étape d'implantation, une étape de mesure, une étape de transfert, une étape de détection et une étape de signalisation.

Au cours de l'étape d'implantation, l'ancre 15 et le dispositif de centralisation 20 sont implantés dans le corps du patient P. Le deuxième dispositif 25 n'est pas implanté dans le corps du patient P.

Au cours de l'étape de mesure, chaque capteur 117 mesure des valeurs du paramètre. Par exemple, le capteur 117 mesure périodiquement des valeurs du paramètre correspondant avec une période prédéterminée.

Les valeurs mesurées sont mémorisées dans la première mémoire 75.

Les valeurs mesurées sont mémorisées dans la première mémoire 75 pour une durée supérieure ou égale à une heure, par exemple supérieure ou égale à une journée, par exemple supérieure ou égale à une semaine, par exemple supérieure ou égale à un mois.

Au cours de l'étape de transfert, les valeurs mesurées sont transférées, par le dispositif de centralisation 20, au deuxième dispositif 25. Par exemple, l'étape de transfert est mise en œuvre lorsque le patient P se rend au cabinet de son médecin attitré.

En variante, l'étape de transfert est mise en œuvre périodiquement lorsque le patient P se trouve à son domicile, par exemple une fois par jour. Selon une variante, lorsque le patient porte sur lui le deuxième dispositif 25, par exemple lorsque le deuxième dispositif 25 est intégré à un téléphone mobile, l'étape de transfert est mise en œuvre avec une période inférieure ou égale à une heure.

Par exemple, grâce aux valeurs de différence de potentiel mesurées, le deuxième dispositif 25 affiche un électrocardiogramme du patient P.

Après chaque étape de transfert, la première mémoire 75 est effacée. En particulier, les valeurs de paramètres mémorisées sont effacées.

Au cours de l'étape de détection, le deuxième contrôleur 135 détecte le phénomène physiologique à partir des valeurs mesurées.

Selon une variante le deuxième contrôleur 135 calcule des données d'exploitation à partir des valeurs mesurées et détecte le phénomène physiologique à partir des données d'exploitation. Les données d'exploitation sont des données dérivées des valeurs mesurées. Par exemple, un rythme cardiaque est un exemple de données d'exploitation calculées à partir de mesures d'une différence de potentiel entre deux électrodes ou à partir de valeurs d'accélération du dispositif de centralisation 20. Un intervalle de temps entre deux ondes de polarisation d'une oreillette du cœur est un autre exemple de données d'exploitation, et une variation de cet intervalle de temps est un autre exemple de données d'exploitation. Un coefficient de corrélation obtenu par un ajustement d'un ensemble de valeurs mesurées avec un ensemble de valeurs de référence ou une fonction de référence est un autre exemple de données d'exploitation.

Par exemple, le deuxième contrôleur 135 compare chaque valeur mesurée ou chaque valeur de données d'exploitation à un seuil prédéterminé et détecte le phénomène physiologique si une des valeurs mesurées est supérieure ou égale au seuil prédéterminé.

Selon un autre exemple, le phénomène physiologique est détecté si plusieurs valeurs mesurées ou plusieurs valeurs de données d'exploitation sont supérieures ou égales à un seuil prédéterminé pendant une période de temps prédéterminée. Selon une variante, le deuxième contrôleur 135 détecte le phénomène physiologique si une ou plusieurs des valeurs mesurées ou des valeurs de données d'exploitation est supérieure ou égale au seuil prédéterminé, ou encore si une ou plusieurs des valeurs mesurées ou des valeurs de données d'exploitation n'est pas comprise dans une plage de données encadrée par deux seuils prédéterminés.

Selon un autre exemple, le phénomène physiologique est détecté si plusieurs valeurs mesurées ou plusieurs valeurs de données d'exploitation appartiennent à une région prédéterminée de l'espace 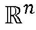 , où 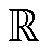 est l'ensemble des réels et n représente le nombre de valeurs. Selon une variante, le deuxième contrôleur 135 détecte le phénomène physiologique si une ou plusieurs des valeurs mesurées ou des valeurs de données d'exploitation appartiennent à une région prédéterminée de l'espace 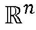 .

Les seuils prédéterminés ou la région prédéterminée de l'espace 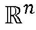 sont, par exemple, déterminés par une méthode d'apprentissage automatique. Les méthodes d'apprentissage automatique sont également appelées « apprentissage machine ». Par exemple, le système implantable enregistre les valeurs mesurées pendant que le patient subit des examens plus classiques (électrocardiogramme externe ou examens morphologiques, par exemple échographiques, éventuellement pratiqués lors d'épreuves fonctionnelles, par exemple des épreuves d'effort). Ces examens plus classiques sont choisis pour permettre de poser un diagnostic, et une méthode d'apprentissage machine permet de déterminer des combinaisons des valeurs mesurées par le système implantable qui correspondent au phénomène physiologique ou pathologique étudié. Dans la vie de tous les jours, lorsque les valeurs mesurées vérifieront à nouveau ces caractéristiques, le deuxième contrôleur 135 détectera le phénomène physiologique ou pathologique.

Selon une variante, le deuxième contrôleur 135 détecte le phénomène physiologique à partir de valeurs mesurées par au moins deux capteurs 117 distincts.

Selon un mode de réalisation, le deuxième contrôleur 135 détecte une fibrillation auriculaire à partir des valeurs d'accélération et/ou des valeurs de tension électrique mesurées. Le deuxième contrôleur 135 détecte, par exemple, la fibrillation auriculaire à partir d'une analyse des valeurs de tension électrique pour détecter la présence ou l'absence, dans l'électrocardiogramme, d'un signal représentatif d'une onde P de polarisation de l'oreillette du cœur. En variante, l'analyse de la variation de l'intervalle de temps entre deux ondes R permet de détecter une fibrillation auriculaire.

L'étape de signalisation est alors mise en œuvre.

Au cours de l'étape de signalisation, le deuxième contrôleur 135 commande l'émission, par l'interface homme/machine 140, d'un signal d'alerte à destination du patient P et/ou de son médecin.

Le signal d'alerte informe le patient P et/ou son médecin que le phénomène physiologique s'est produit. Par exemple, le signal d'alerte contient une date de survenue du phénomène physiologique, une fréquence d'apparition du phénomène physiologique, une durée du phénomène physiologique, ou encore un rythme cardiaque du patient P au cours du phénomène physiologique.

Le signal d'alerte est, par exemple, transmis simultanément à un organisme de secours. Par exemple, le signal d'alerte est envoyé à un organisme de secours via un réseau de téléphonie sans fil. En variante, le signal d'alerte est envoyé par le réseau Internet.

Du fait que le dispositif de centralisation 20 est dans l'estomac, le remplacement de la première réserve d'énergie électrique 90 est aisé et peut, par exemple, être effectué par voie endoscopique depuis l'œsophage, de manière simple et rapide. En outre, le remplacement de la première réserve d'énergie électrique 90 pose peu de risques d'infection puisque aucune incision n'est réalisée.

L'utilisation des attracteurs 110 rend encore plus simple la mise en place de la première réserve d'énergie électrique 90, même sans endoscopie, puisqu'il suffit alors que le patient P avale la première réserve d'énergie électrique 90.

En outre, le système implantable 10 ne suppose pas que le patient P porte en permanence des moyens de stockage d'énergie électrique à l'extérieur de son corps, ni que des conducteurs électriques disgracieux débouchent hors du corps du patient P. Le système implantable 10 est donc peu contraignant pour le patient.

La partie supérieure de l'estomac 30 est proche du cœur C, et les contractions du cœur C provoquent donc un déplacement du dispositif de centralisation 20. Grâce au positionnement d'un capteur d'accélération 117 dans l'estomac, une fibrillation auriculaire est alors efficacement détectée, non seulement par une mesure électrique de l'activité du cœur mais également par la mesure de l'accélération du dispositif de centralisation 20.

Le système implantable améliore donc la sécurité du patient P.

En outre, le positionnement du dispositif de centralisation 20 dans l'estomac permet, là encore, de diminuer les contraintes pour le patient P.

En outre, le traitement des données acquises par les capteurs 117 est effectué par le deuxième dispositif 25, qui est situé en-dehors du corps du patient. La consommation électrique du dispositif de centralisation 20 est alors limitée, ce qui diminue la fréquence à laquelle l'alimentation électrique du dispositif de centralisation 20 doit être changée. Les contraintes pour le patient P sont donc ici encore limitées.

Selon une variante du premier exemple, l'étape de mesure est mise en œuvre pendant des plages temporelles non-continues. Par exemple, des valeurs sont mesurées pendant des premières plages temporelles séparées par des deuxièmes plages temporelles pendant lesquelles aucune valeur n'est mesurée. Selon un mode de réalisation, les premières plages temporelles présentent une durée de 30 secondes, et les deuxièmes plages temporelles présentent une durée de 30 minutes.

Ainsi, le premier dispositif 20 est mis en sommeil pendant une grande proportion du temps. La consommation énergétique du système implantable 10 est donc réduite. Ce mode de réalisation est particulièrement adapté à la détection de phénomènes physiologiques dont l'évolution est lente, tels que les insuffisances cardiaques.

Le premier exemple de système implantable 10 a été donné pour le cas de la détection d'une fibrillation auriculaire du patient P. En variante, le système implantable 10 peut aussi permettre de détecter d'autres pathologies cardio-vasculaires, par exemple une insuffisance cardiaque. Cependant, il est à noter que le positionnement du dispositif de centralisation dans l'estomac 30 du patient P permet de mesurer des paramètres d'une grande variété d'organes C. Le système implantable 10 est donc susceptible d'être adapté à la détection d'un grand nombre de phénomènes physiologiques distincts.

Par exemple, le phénomène physiologique est un trouble respiratoire. Une broncho-pneumopathie chronique obstructive est un exemple de trouble respiratoire.

En variante, le phénomène physiologique est un emphysème.

Selon une autre variante, le phénomène physiologique est une épilepsie.

Selon une autre variante, le phénomène physiologique est un trouble de la nutrition.

Selon une autre variante, le phénomène physiologique est une apnéedu sommeil. Par exemple, le capteur 117 est propre à détecter une contraction du diaphragme et le deuxième contrôleur 135 est configuré pour détecter une apnée du sommeil si une période de temps sans contraction du diaphragme présente une durée supérieure ou égale à un seuil prédéterminé.

Selon une autre variante, le phénomène physiologique est un trouble de la déglutition, ou encore un trouble de l'alimentation tel qu'une hydratation insuffisante.

De plus, de nombreux types de capteurs 117 sont susceptibles d'être utilisés.

Par exemple, le capteur 117 est un émetteur/récepteur de lumière comportant au moins une source lumineuse et au moins un détecteur de rayonnements lumineux. Par exemple, le capteur 117 comporte deux sources lumineuses. Selon un mode de réalisation, le capteur 117 comporte alors deux détecteurs de rayonnement lumineux.

La ou les sources lumineuses sont configurées pour illuminer au moins une portion d'un organe du patient P avec un rayonnement lumineux. Par exemple, chaque source lumineuse est configurée pour illuminer une portion du muscle cardiaque. La portion est, par exemple, une portion d'une cavité cardiaque.

L'organe illuminé est, en variante, un vaisseau sanguin tel que l'aorte du patient P.

En variante, le vaisseau sanguin est la veine cave.

Selon une autre variante, l'organe illuminé est la paroi gastrique.

Selon une autre variante, l'organe illuminé est le diaphragme.

Chaque rayonnement lumineux présente une longueur d'onde. Par exemple, au moins une longueur d'onde est choisie pour son niveau d'absorption ou de réflexion par certaines molécules. Par exemple, au moins un rayonnement lumineux présente une longueur d'onde visible. Une longueur d'onde égale à 660 nanomètres est un exemple de longueur d'onde visible.

Selon une variante, le rayonnement lumineux d'au moins une source lumineuse est un rayonnement lumineux infrarouge. Par exemple, le rayonnement infrarouge présente une longueur d'onde égale à 950 nanomètres.

Selon un mode de réalisation, une longueur d'onde est égale à 660 nanomètres et une autre longueur d'onde est égale à 950 nanomètres. Ces longueurs d'onde sont absorbées et/ou réfléchies différemment par l'hémoglobine non saturée (notée Hb) et l'hémoglobine saturée (notée HbO₂), et permettent donc une bonne évaluation du rapport entre ces deux molécules.

Le détecteur est configuré pour mesurer une valeur d'un taux de réflexion de chaque rayonnement lumineux sur l'organe illuminé. Par exemple, le détecteur comporte une photodiode.

En variante, le détecteur est configuré pour mesurer un taux d'absorption de chaque rayonnement lumineux.

Le deuxième contrôleur 135 est configuré pour recevoir du détecteur les valeurs de taux de réflexion mesuré, et pour calculer un taux d'oxygénation du sang circulant dans l'organe illuminé à partir des valeurs reçues. Par exemple, le deuxième contrôleur 135 est configuré pour calculer un taux d'oxygénation du sang capillaire circulant dans la paroi gastrique. En variante, le deuxième contrôleur 135 est configuré pour calculer un taux d'oxygénation du sang capillaire circulant dans le muscle cardiaque, ou du sang présent dans les cavités cardiaques, ou du sang présent dans l'aorte ou dans la veine cave.

Le phénomène physiologique que le système implantable 10 est propre à détecter est alors une baisse de la saturation en oxygène de l'hémoglobine du patient P. La baisse est, par exemple, causée par une pathologie cardiaque ou respiratoire.

Selon une autre variante, au moins un capteur 117 est un capteur d'un marqueur biologique d'un fluide corporel F du patient P. Le fluide corporel F est, par exemple, le contenu de l'estomac ou le liquide extra-cellulaire au niveau de la paroi de l'estomac où le premier dispositif 20 est implanté.

Selon une autre variante, le dispositif de centralisation 20 comporte un premier cathéter configuré pour conduire un fluide corporel F du patient P depuis l'organe C jusqu'au capteur 117. Par exemple, l'organe C est le péritoine, et le fluide F est le fluide péritonéal.

Le marqueur biologique est, par exemple, le glucose.

En variante, le marqueur biologique est un ion présent dans le fluide corporel F. Par exemple, le capteur 117 est propre à mesurer le pH du fluide corporel F. En particulier, le capteur 117 est propre à mesurer le pH du contenu gastrique ou du liquide péritonéal du patient P.

Le capteur 117 est alors propre à mesurer un taux du marqueur biologique dans le fluide corporel F. Par exemple, le capteur 117 est propre à mesurer un taux de glucose dans le liquide péritonéal, et le premier calculateur 45 est propre à estimer une valeur de glycémie du patient P.

Selon une autre variante, au moins un capteur 117 est configuré pour estimer une orientation du dispositif de centralisation 20 par rapport à la verticale. Le premier contrôleur 45 est alors configuré pour détecter une position du patient P. En particulier, le premier contrôleur 45 est configuré pour détecter une position couchée, une position assise ou une position intermédiaire entre les positions couchée et assise du patient P.

Par exemple, le capteur 117 comporte au moins un élément de la liste formée par : un gyroscope, un magnétomètre et un accéléromètre.

Selon une autre variante, au moins un capteur 117 est propre à mesurer une température corporelle du patient P.

Selon une autre variante, au moins un capteur 117 est un microphone. Le capteur 117 est configuré pour mesurer un bruit émis par l'organe C. Le deuxième contrôleur 135 est propre à détecter le phénomène physiologique à partir de l'analyse des bruits mesurés. Par exemple, le deuxième contrôleur 135 est propre à détecter un trouble du rythme cardiaque, une anomalie d'une valve du cœur, une pathologie pulmonaire ou encore un trouble de la digestion à partir des bruits mesurés.

Selon une autre variante, au moins un capteur 117 est un émetteur/récepteur d'ultrasons. Par exemple, le capteur 117 est propre à émettre au moins un faisceau d'ultrasons et à mesurer un paramètre d'un faisceau réfléchi sur tout ou partie du cœur du patient P. Le paramètre du faisceau est, par exemple, un taux de réflexion, ou encore un déphasage.

Le deuxième contrôleur 135 est, par exemple, propre à calculer une dimension ou une amplitude de contraction du cœur du patient P à partir des mesures fournies par le capteur 117. En particulier, le deuxième contrôleur 135 est configuré pour calculer une fraction d'éjection du cœur à partir des dimensions ou des amplitudes de contraction mesurées.

En variante, le faisceau émis est réfléchi sur un vaisseau sanguin tel que l'aorte et les données d'exploitation comprennent une variation du diamètre du vaisseau sanguin. Selon une variante, le vaisseau est la veine cave.

Selon un mode de réalisation le capteur 117 est configuré pour évaluer un débit sanguin ou une pression sanguine dans un vaisseau sanguin par effet Doppler.

Un capteur de pression est un autre exemple de capteur 117.

Selon une autre variante, le premier dispositif 20 comporte plusieurs capteurs 117, et le deuxième contrôleur 135 est configuré pour détecter le phénomène physiologique à partir de valeurs mesurées par au moins deux capteurs 117. Selon une autre variante, le deuxième contrôleur 135 est propre à détecter au moins deux phénomènes physiologiques distincts à partir des valeurs mesurées par le ou les capteurs 117.

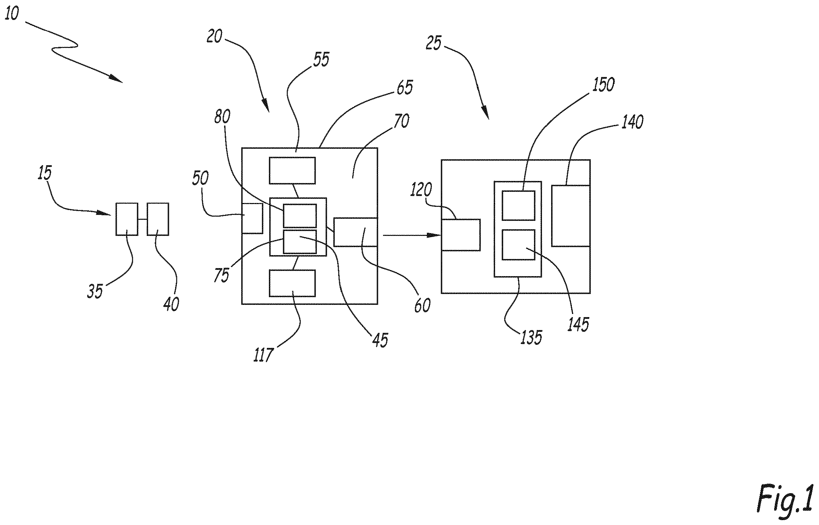

Un deuxième exemple de système implantable 10 va maintenant être décrit. Les éléments identiques au premier exemple de système implantable 10 de la figure 1 ne sont pas décrits à nouveau. Seules les différences sont mises en évidence.

Le système implantable 10 comporte deux deuxièmes dispositifs 25.

L'un des deuxièmes dispositifs 25 est un dispositif d'affichage tel que décrit précédemment. L'autre deuxième dispositif 25 sera désigné par la suite par l'expression « dispositif de mesure ». Afin de les distinguer l'un de l'autre, les deux deuxièmes dispositifs 25 seront désignés dans la suite du deuxième exemple par les expressions « dispositif d'affichage » et « dispositif de mesure » respectivement.

Le dispositif de mesure 25 est implanté, dans sa position de fonctionnement, dans le corps du patient P. En particulier, le dispositif de mesure 25 est implanté hors de l'estomac 30 du patient P.

Selon une variante, le dispositif de mesure 25 n'est pas implanté dans le corps du patient P mais est porté par le patient P. Par exemple, le dispositif de mesure 25 est fixé autour d'un membre du patient P par une lanière. Il est à noter que d'autres positions de fonctionnement et d'autres modes de fixation sont envisageables.

Le dispositif de mesure 25 ne comporte pas d'interface homme-machine 140.

Le dispositif de mesure 25 comprend au moins un capteur 117.

Le deuxième émetteur/récepteur 120 du dispositif de mesure 25 est configuré pour transmettre au premier émetteur/récepteur 60 les valeurs mesurées.

La première mémoire 75 est, en outre, configurée pour stocker les valeurs mesurées par le ou les capteurs du dispositif de mesure 25.

Le premier émetteur/récepteur 60 est configuré pour transmettre au dispositif d'affichage 25 les valeurs mesurées par le ou les capteurs 117 du dispositif de mesure 25.

Le deuxième contrôleur 135 est alors configuré pour détecter au moins un phénomène physiologique à partir d'au moins une des valeurs mesurées par le ou les capteurs 117 du dispositif de mesure 25.

Le système implantable 10 est alors susceptible d'être utilisé pour la détection de phénomènes physiologiques pour lesquels le positionnement du capteur 117 dans l'estomac n'est pas favorable.

Selon une variante du deuxième exemple, le dispositif de centralisation 20 ne comporte pas de capteur 117. Seul le dispositif de mesure 25 comporte au moins un capteur 117. Le dispositif de centralisation 20 joue alors un rôle de stockage et de transmission au dispositif d'affichage 25 des valeurs mesurées.

Selon une autre variante, le système implantable 10 comprend au moins deux dispositifs de mesure 25.

Un troisième exemple de système implantable 10 va maintenant être décrit. Les éléments identiques au premier exemple de système implantable 10 ne sont pas décrits à nouveau. Seules les différences sont mises en évidence.

L'alimentation électrique 55 ne comporte pas de troisième connecteur 85 ni de réserve d'énergie électrique 90.

L'alimentation électrique 55 comporte un générateur d'énergie électrique. Il est entendu par « générateur d'énergie électrique » que le générateur d'énergie électrique n'est pas configuré pour être chargé en énergie électrique par un courant électrique.

Le générateur d'énergie électrique est propre à générer au moins un courant électrique par réaction d'au moins une espèce chimique présente dans le corps du patient P. Plus précisément, le générateur d'énergie électrique est propre à générer le courant d'alimentation C.

Par exemple, le générateur d'énergie électrique comprend deux électrodes, les électrodes baignant dans les sucs gastriques du patient P lorsque le dispositif de centralisation 20 est dans la position de fixation. En variante, les électrodes du générateur d'énergie électrique sont prévues pour baigner dans l'intestin du patient P lorsque le dispositif de centralisation 20 est dans la position de fixation.

Chaque électrode comporte au moins une enzyme. En variante, chaque électrode comporte au moins un micro-organisme. Par exemple, chaque électrode du générateur d'énergie électrique comporte un conducteur électrique recouvert de l'enzyme ou du micro-organisme, l'ensemble ainsi formé étant entouré d'une membrane. La membrane est, par exemple, configurée pour être traversée par certaines espèces chimiques naturellement présentes dans l'estomac ou l'intestin du patient P.

Lorsque les électrodes du générateur d'énergie électrique sont immergées dans les sucs gastriques ou dans le liquide intestinal, l'une des électrodes joue le rôle d'anode dans une réaction d'oxydo-réduction mettant en jeu une première espèce chimique. Simultanément, l'autre électrode joue le rôle de cathode dans une réaction d'oxydoréduction mettant en jeu une deuxième espèce chimique.

Par l'oxydation et la réduction simultanée de la première espèce chimique et de la deuxième espèce chimique, une tension électrique apparaît entre les deux conducteurs électriques. Le courant d'alimentation C est alors généré.

La première espèce chimique est, par exemple, le glucose. La deuxième espèce chimique est, par exemple, l'oxygène.

Le troisième exemple de système implantable 10 ne nécessite pas de charger électriquement une réserve d'énergie électrique 90 ni de faire pénétrer la réserve d'énergie électrique 90 dans le corps du patient P.

Les contraintes pour le patient P sont, là encore, réduites.

Selon un quatrième exemple, le générateur d'énergie électrique est propre à générer au moins un courant électrique par conversion d'une énergie mécanique en énergie électrique. En particulier, le générateur d'énergie électrique est propre à générer au moins un courant électrique à partir des mouvements de l'estomac 30.

Selon un cinquième exemple, le premier contrôleur 45 détecte le phénomène physiologique à partir des valeurs mesurées. Seul un message comprenant les résultats de la détection est transmis au deuxième dispositif 25.

Dans la description ci-dessus, les fonctions du système implantable 10 ont été séparées en plusieurs exemples pour faciliter leur compréhension par le lecteur. Cependant, il est à noter que les exemples précédents sont susceptibles d'être combinés pour générer de nouveaux modes de réalisation.

Le système implantable 10 est particulièrement adapté à la détection d'une insuffisance cardiaque. Dans ce cas, au moins un des capteurs 117 est choisi parmi l'ensemble formé de : un capteur mesurant une différence de potentiel électrique, un accéléromètre, un émetteur/récepteur d'ultrasons, un émetteur/récepteur de lumière et un microphone. Par exemple, deux capteurs 117 intégrés au dispositif de centralisation 20 sont choisis parmi l'ensemble formé de : un capteur mesurant une différence de potentiel électrique, un accéléromètre, un émetteur/récepteur d'ultrasons, un émetteur/récepteur de lumière et un microphone.

Le deuxième contrôleur 135 est alors configuré pour détecter une insuffisance cardiaque à partir de valeurs mesurées par les capteurs 117 et/ou à partir de données d'exploitation calculées à partir des valeurs mesurées par les capteurs 117.

Par exemple, le deuxième contrôleur 135 est configuré pour détecter une insuffisance cardiaque à partir de valeurs de potentiel électrique.

Par exemple, le deuxième contrôleur 135 est configuré pour détecter une insuffisance cardiaque à partir de valeurs d'accélération.

Selon un mode de réalisation, le deuxième contrôleur 135 est configuré pour détecter une insuffisance cardiaque à partir d'une combinaison de valeurs de potentiel électrique, d'accélération et d'orientation du patient.

Par exemple, le deuxième contrôleur 135 est configuré pour détecter une insuffisance cardiaque à partir de valeurs de taux d'oxygénation du sang ou d'une fraction d'éjection du cœur.

Par exemple, le deuxième contrôleur 135 est configuré pour détecter une insuffisance cardiaque à partir d'une combinaison de valeurs mesurées par tous les capteurs 117 et de valeurs de toutes les données d'exploitation.

Si un des capteurs 117 est un émetteur/récepteur d'ultrasons, le deuxième contrôleur 135 calcule une fraction d'éjection du cœur à partir des variations des dimensions du cœur et détecte l'insuffisance cardiaque si la fraction d'éjection est inférieure ou égale à un seuil correspondant.

En variante, l'insuffisance cardiaque est détectée à partir d'une analyse des bruits du cœur ou du poumon.

De plus, la description ci-dessus a été donnée dans le cas où l'ancre 15 et le dispositif de centralisation 20 forment deux dispositifs séparés. Il est à noter que le dispositif de centralisation 20 et l'ancre 15 sont susceptibles de former un seul dispositif, l'ancre 15 et le dispositif de centralisation 20 n'étant alors pas séparables l'un de l'autre. Par exemple, l'ancre 15 est venue de matière avec le boîtier 65 du dispositif de centralisation 20.

Selon encore un exemple, la tête 35 comporte au moins une semelle située à l'extérieur de l'estomac 30. Par exemple, la tête 35 comporte deux semelles.

Chaque semelle est configurée pour venir en appui contre la face extérieure de la paroi de l'estomac 30 et pour être reliée au dispositif implantable 20 de manière à exercer une force tendant à plaquer le dispositif implantable 20 contre la face intérieure de la paroi de l'estomac 30. Selon une variante, chaque semelle est configurée pour être placée entre le feuillet viscéral et le feuillet pariétal du péritoine et pour venir en appui contre le feuillet viscéral pour plaquer le dispositif implantable 20 contre la face intérieure de la paroi de l'estomac 30.

Chaque semelle est, par exemple, une plaque. En variante, chaque semelle comporte un treillis de fils tendus sur un cadre, en particulier un cadre souple propre à être plié et inséré dans un endoscope ou une aiguille creuse.

Le premier connecteur 40 comporte, par exemple, un ou plusieurs anneaux solidaires du boîtier 65. Chaque semelle est, par exemple, fixée au dispositif implantable 20 par un ou plusieurs fils fixés à un ou plusieurs des anneaux.

La fixation par une ou plusieurs semelles permet de répartir la pression exercée par le dispositif implantable sur une plus grande surface de la paroi de l'estomac 30 et de diminuer donc la pression ainsi exercée. En outre, ce mode de fixation ne suppose pas de générer dans la paroi stomacale un pli qui réduit le volume de l'estomac, qui est susceptible de générer des tensions dans l'ancre qui y est fixée. Puisque les forces exercées sur la paroi stomacale sont réduites, les risques d'apparition d'une réaction inflammatoire de la muqueuse gastrique sont limités.

## Revendications

1. Système implantable (10) comprenant :
- un premier dispositif (20), appelé dispositif de centralisation, propre à être fixé dans une position de fixation à une paroi de l'estomac (30) d'un patient (P), le dispositif de centralisation (20) étant accueilli dans l'estomac (30) lorsque le dispositif de centralisation (20) est dans la position de fixation, et
- au moins un deuxième dispositif (25),
le système implantable (10) étant **caractérisé en ce que** le dispositif de centralisation (20) comprend un contrôleur (45), une alimentation électrique (55) et un émetteur/récepteur (60) propre à permettre la communication entre le dispositif de centralisation (20) et le deuxième dispositif (25) lorsque le deuxième dispositif (25) est dans une position de fonctionnement, le deuxième dispositif (25) étant situé en-dehors du corps du patient (P) lorsque le deuxième dispositif (25) est dans la position de fonctionnement, dans lequel :
- le dispositif de centralisation (20) comporte un capteur (117) propre à mesurer une valeur d'un paramètre physiologique du patient (P), l'émetteur/récepteur (60) étant configuré pour transmettre au deuxième dispositif (25) les valeurs mesurées, le deuxième dispositif (25) considéré étant propre à détecter un phénomène physiologique du patient (P) à partir d'au moins une des valeurs mesurées et la communication entre le dispositif de centralisation (20) et l'au moins un deuxième dispositif (25) s'effectuant par radiofréquence.

2. Système implantable (10) comprenant :
- un premier dispositif (20), appelé dispositif de centralisation, propre à être fixé dans une position de fixation à une paroi de l'estomac (30) d'un patient (P), le dispositif de centralisation (20) étant accueilli dans l'estomac (30) lorsque le dispositif de centralisation (20) est dans la position de fixation,
- le système implantable (10) comprenant deux deuxièmes dispositifs (25),
le système implantable (10) étant **caractérisé en ce que** le dispositif de centralisation (20) comprend un contrôleur (45), une alimentation électrique (55) et un émetteur/récepteur (60) propre à permettre la communication entre le dispositif de centralisation (20) et chacun des deuxièmes dispositifs (25) lorsque chacun des deuxièmes dispositifs (25) est dans une position de fonctionnement, au moins un des deuxièmes dispositif (25) étant situé en-dehors du corps du patient (P) lorsque cet au moins un des deuxièmes dispositif (25) est dans la position de fonctionnement, dans lequel :
- l'un des deuxièmes dispositifs autrement appelé dispositif de mesure (25) comporte au moins un capteur (117) propre à mesurer une valeur d'un paramètre physiologique du patient (P), l'émetteur/récepteur (60) étant configuré pour recevoir du dispositif de mesure (25) comportant un capteur (117) les valeurs mesurées et pour transmettre les valeurs mesurées à l'autre deuxième dispositif, appelé dispositif d'affichage (25), celui-ci étant propre à détecter un phénomène physiologique du patient (P) à partir d'au moins une des valeurs reçues de l'émetteur/récepteur (60), la communication entre le dispositif de centralisation (20) et chacun des deuxièmes dispositifs (25) s'effectuant par radiofréquence.

3. Système implantable (10) selon l'une quelconque des revendications 1 ou 2, dans lequel le contrôleur (45) comporte une mémoire (75) propre à stocker les valeurs mesurées pendant une durée supérieure ou égale à un jour.

4. Système implantable (10) selon l'une quelconque des revendications 1 à 3, dans lequel le phénomène physiologique est une pathologie cardiaque.

5. Système implantable (10) selon l'une quelconque des revendications 1 à 4, dans lequel le capteur (117) est un émetteur/récepteur d'ultrasons.

6. Système implantable (10) selon l'une quelconque des revendications 3 ou 4 en combinaison avec la revendication 1, dans lequel le capteur (117) est un accéléromètre propre à mesurer des valeurs d'une accélération du dispositif de centralisation (20) et le deuxième dispositif (25) est configuré pour calculer un paramètre physiologique du cœur du patient (P) à partir des valeurs d'accélération mesurées.

7. Système implantable (10) selon l'une quelconque des revendications 3 ou 4 en combinaison avec la revendication 2, dans lequel le capteur (117) est un accéléromètre propre à mesurer des valeurs d'une accélération du dispositif de centralisation (20) et le dispositif de mesure (25) est configuré pour calculer un paramètre physiologique du cœur du patient (P) à partir des valeurs d'accélération mesurées.

8. Système implantable (10) selon l'une quelconque des revendications 1 à 4, dans lequel le capteur (117) est propre à mesurer une différence de potentiel électrique, une accélération, un bruit, une orientation, un pH ou une température.

9. Système implantable (10) selon l'une quelconque des revendications 1 à 4, dans lequel le dispositif de centralisation (20) comprend un cathéter propre à conduire un fluide corporel (F) du patient (P) au capteur (117), le capteur (117) étant propre à mesurer un taux d'un marqueur biologique dans le fluide corporel (F).

10. Système implantable (10) selon l'une quelconque des revendications 1 à 4, dans lequel le capteur (117) comporte au moins une source lumineuse et au moins un détecteur de rayonnement lumineux, la source lumineuse étant configurée pour illuminer au moins une portion d'un organe du patient (P) avec un rayonnement lumineux, le détecteur étant configuré pour mesurer une valeur d'un taux de réflexion du rayonnement lumineux, le contrôleur (45) étant configuré pour calculer un taux d'oxygénation du sang circulant dans l'organe illuminé par la source lumineuse, à partir du taux de réflexion.

11. Système implantable (10) selon l'une quelconque des revendications 3 à 10 en combinaison avec la revendication 1, comprenant une pluralité de capteurs (117), dans lequel le deuxième dispositif (25) est propre à détecter le phénomène physiologique à partir de valeurs d'au moins deux capteurs (117).

12. Système implantable (10) selon l'une quelconque des revendications 3 à 10 en combinaison avec la revendication 2, comprenant une pluralité de capteurs (117), dans lequel le dispositif de mesure (25) est propre à détecter le phénomène physiologique à partir de valeurs d'au moins deux capteurs (117).

13. Système implantable (10) selon l'une quelconque des revendications 1 à 12, dans lequel l'alimentation électrique (55) comporte une réserve (90) d'énergie électrique amovible et un connecteur (85) propre à accueillir la réserve d'énergie électrique (90), la réserve d'énergie électrique (90) étant propre à alimenter électriquement le contrôleur (45) lorsque la réserve d'énergie électrique (90) est connectée électriquement au connecteur (85) dans une position de connexion et de préférence étant configurée pour être avalée par le patient (P) et pour se déplacer spontanément jusqu'à la position de connexion depuis une position de déconnexion dans laquelle la réserve d'énergie électrique (90) est accueillie dans l'estomac (30) du patient (P) et est déconnectée du connecteur (85).

14. Système implantable (10) selon l'une quelconque des revendications 1 à 13, dans lequel l'alimentation électrique (55) comporte un générateur d'énergie électrique propre à générer un courant électrique par réaction d'au moins une espèce chimique présente dans le corps du patient (P), notamment le glucose.

15. Système implantable (10) selon l'une quelconque des revendications 1 à 14, dans lequel l'alimentation électrique (55) comporte un générateur d'énergie électrique propre à générer un courant électrique par conversion d'énergie mécanique en énergie électrique.

## Patentansprüche

1. Implantierbares System (10), das Folgendes beinhaltet:
- eine erste Vorrichtung (20), als Zentralisierungsvorrichtung bezeichnet, die dazu geeignet ist, an einer Wand des Magens (30) eines Patienten (P) in einer Befestigungsposition befestigt zu sein, wobei die Zentralisierungsvorrichtung (20) in dem Magen (30) aufgenommen ist, wenn sich die Zentralisierungsvorrichtung (20) in der Befestigungsposition befindet, und
- mindestens eine zweite Vorrichtung (25),
wobei das implantierbare System (10) **dadurch gekennzeichnet ist, dass** die Zentralisierungsvorrichtung (20) Folgendes beinhaltet: eine Steuereinheit (45), eine elektrische Versorgungseinheit (55) und einen Sender/Empfänger (60), der dazu geeignet ist, die Kommunikation zwischen der Zentralisierungsvorrichtung (20) und der zweiten Vorrichtung (25) zu ermöglichen, wenn sich die zweite Vorrichtung (25) in einer Betriebsposition befindet, wobei sich die zweite Vorrichtung (25) außerhalb des Körpers des Patienten (P) befindet, wenn sich die zweite Vorrichtung (25) in der Betriebsposition befindet, wobei:
- die Zentralisierungsvorrichtung (20) einen Sensor (117) umfasst, der dazu geeignet ist, einen Wert eines physiologischen Parameters des Patienten (P) zu messen, wobei der Sender/Empfänger (60) dazu konfiguriert ist, die gemessenen Werte an die zweite Vorrichtung (25) zu übertragen, wobei die betrachtete zweite Vorrichtung (25) dazu geeignet ist, basierend auf mindestens einem der gemessenen Werte ein physiologisches Phänomen des Patienten (P) zu detektieren, und wobei die Kommunikation zwischen der Zentralisierungsvorrichtung (20) und der mindestens einen zweiten Vorrichtung (25) per Radiofrequenz erfolgt.

2. Implantierbares System (10), das Folgendes beinhaltet:
- eine erste Vorrichtung (20), als Zentralisierungsvorrichtung bezeichnet, die dazu geeignet ist, an einer Wand des Magens (30) eines Patienten (P) in einer Befestigungsposition befestigt zu sein, wobei die Zentralisierungsvorrichtung (20) in dem Magen (30) aufgenommen ist, wenn sich die Zentralisierungsvorrichtung (20) in der Befestigungsposition befindet,
- wobei das implantierbare System (10) zwei zweite Vorrichtungen (25) beinhaltet,
wobei das implantierbare System (10) **dadurch gekennzeichnet ist, dass** die Zentralisierungsvorrichtung (20) Folgendes beinhaltet: eine Steuereinheit (45), eine elektrische Versorgungseinheit (55) und einen Sender/Empfänger (60), der dazu geeignet ist, die Kommunikation zwischen der Zentralisierungsvorrichtung (20) und jeder der zweiten Vorrichtungen (25) zu ermöglichen, wenn sich jede der zweiten Vorrichtungen (25) in einer Betriebsposition befindet, wobei sich mindestens eine der zweiten Vorrichtungen (25) außerhalb des Körpers des Patienten (P) befindet, wenn sich diese mindestens eine der zweiten Vorrichtungen (25) in der Betriebsposition befindet, wobei:
- eine der zweiten Vorrichtungen, auch als Messvorrichtung (25) bezeichnet, mindestens einen Sensor (117) umfasst, der dazu geeignet ist, einen Wert eines physiologischen Parameters des Patienten (P) zu messen, wobei der Sender/Empfänger (60) dazu konfiguriert ist, von der Messvorrichtung (25), die einen Sensor (117) umfasst, die gemessenen Werte zu empfangen und die gemessenen Werte an die andere zweite Vorrichtung, die als Anzeigevorrichtung (25) bezeichnet wird, zu übertragen, wobei diese dazu geeignet ist, basierend auf mindestens einem der von dem Sender/Empfänger (60) empfangenen Werte ein physiologisches Phänomen des Patienten (P) zu detektieren, wobei die Kommunikation zwischen der Zentralisierungsvorrichtung (20) und jeder der zweiten Vorrichtungen (25) per Radiofrequenz erfolgt.

3. Implantierbares System (10) nach einem der Ansprüche 1 oder 2, wobei die Steuereinheit (45) einen Speicher (75) umfasst, der dazu geeignet ist, die gemessenen Werte für einen Zeitraum zu speichern, der größer als oder gleich einem Tag ist.

4. Implantierbares System (10) nach einem der Ansprüche 1 bis 3, wobei das physiologische Phänomen eine Herzerkrankung ist.

5. Implantierbares System (10) nach einem der Ansprüche 1 bis 4, wobei der Sensor (117) ein Ultraschallsender/-empfänger ist.

6. Implantierbares System (10) nach einem der Ansprüche 3 oder 4 in Kombination mit Anspruch 1, wobei der Sensor (117) ein Beschleunigungsmesser ist, der dazu geeignet ist, Werte einer Beschleunigung der Zentralisierungsvorrichtung (20) zu messen, und wobei die zweite Vorrichtung (25) dazu konfiguriert ist, basierend auf den gemessenen Beschleunigungswerten einen physiologischen Parameter des Herzens des Patienten (P) zu berechnen.

7. Implantierbares System (10) nach einem der Ansprüche 3 oder 4 in Kombination mit Anspruch 2, wobei der Sensor (117) ein Beschleunigungsmesser ist, der dazu geeignet ist, Werte einer Beschleunigung der Zentralisierungsvorrichtung (20) zu messen, und wobei die Messvorrichtung (25) dazu konfiguriert ist, basierend auf den gemessenen Beschleunigungswerten einen physiologischen Parameter des Herzens des Patienten (P) zu berechnen.

8. Implantierbares System (10) nach einem der Ansprüche 1 bis 4, wobei der Sensor (117) dazu geeignet ist, eine elektrische Potenzialdifferenz, eine Beschleunigung, ein Geräusch, eine Ausrichtung, einen pH-Wert oder eine Temperatur zu messen.

9. Implantierbares System (10) nach einem der Ansprüche 1 bis 4, wobei die Zentralisierungsvorrichtung (20) einen Katheter beinhaltet, der dazu geeignet ist, eine Körperflüssigkeit (F) des Patienten (P) zu dem Sensor (117) zu leiten, wobei der Sensor (117) dazu geeignet ist, eine Konzentration eines Biomarkers in der Körperflüssigkeit (F) zu messen.

10. Implantierbares System (10) nach einem der Ansprüche 1 bis 4, wobei der Sensor (117) mindestens eine Lichtquelle und mindestens einen Detektor für eine Lichtstrahlung umfasst, wobei die Lichtquelle dazu konfiguriert ist, mindestens einen Abschnitt eines Organs des Patienten (P) mit einer Lichtstrahlung anzustrahlen, wobei der Detektor dazu konfiguriert ist, einen Wert eines Reflexionsgrades der Lichtstrahlung zu messen, wobei die Steuereinheit (45) dazu konfiguriert ist, basierend auf dem Reflexionsgrad einen Sauerstoffgehalt des Blutes, das in dem durch die Lichtquelle angestrahlten Organ zirkuliert, zu berechnen.

11. Implantierbares System (10) nach einem der Ansprüche 3 bis 10 in Kombination mit Anspruch 1, das eine Vielzahl von Sensoren (117) beinhaltet, wobei die zweite Vorrichtung (25) dazu geeignet ist, basierend auf Werten von mindestens zwei Sensoren (117) das physiologische Phänomen zu detektieren.

12. Implantierbares System (10) nach einem der Ansprüche 3 bis 10 in Kombination mit Anspruch 2, das eine Vielzahl von Sensoren (117) beinhaltet, wobei die Messvorrichtung (25) dazu geeignet ist, basierend auf Werten von mindestens zwei Sensoren (117) das physiologische Phänomen zu detektieren.

13. Implantierbares System (10) nach einem der Ansprüche 1 bis 12, wobei die elektrische Versorgungseinheit (55) eine auswechselbare elektrische Energiereserve (90) und einen Verbinder (85), der dazu geeignet ist, die elektrische Energiereserve (90) aufzunehmen, umfasst, wobei die elektrische Energiereserve (90) dazu geeignet ist, die Steuereinheit (45) elektrisch zu versorgen, wenn die elektrische Energiereserve (90) mit dem Verbinder (85) in einer Verbindungsposition elektrisch verbunden ist, und vorzugsweise dazu konfiguriert ist, von dem Patienten (P) geschluckt zu werden und sich von einer Trennungsposition, in der die elektrische Energiereserve (90) in dem Magen (30) des Patienten (P) aufgenommen ist und von dem Verbinder (85) getrennt ist, spontan bis zu der Verbindungsposition zu bewegen.

14. Implantierbares System (10) nach einem der Ansprüche 1 bis 13, wobei die elektrische Versorgungseinheit (55) einen Erzeuger elektrischer Energie umfasst, der dazu geeignet ist, durch Reaktion mindestens einer chemischen Substanz, die in dem Körper des Patienten (P) vorhanden ist, insbesondere Glukose, einen elektrischen Strom zu erzeugen.

15. Implantierbares System (10) nach einem der Ansprüche 1 bis 14, wobei die elektrische Versorgungseinheit (55) einen Erzeuger elektrischer Energie umfasst, der dazu geeignet ist, durch die Umwandlung mechanischer Energie in elektrische Energie einen elektrischen Strom zu erzeugen.

## Claims

1. Implantable system (10) comprising:
- a first device (20), called the centralizing device, able to be attached in an attachment position to a wall of the stomach (30) of a patient (P), the centralizing device (20) being accommodated in the stomach (30) when the centralizing device (20) is in the attachment position, and
- at least one second device (25),
the implantable system (10) being **characterized in that** the centralizing device (20) comprises a controller (45), an electrical power supply (55) and a transceiver (60) able to allow communication between the centralizing device (20) and the second device (25) when the second device (25) is in a working position, the second device (25) being located outside the body of the patient (P) when the second device (25) is in the working position, wherein:
- the centralizing device (20) comprises a sensor (117) able to measure a value of a physiological parameter of the patient (P), the transceiver (60) being configured to transmit, to the second device (25), the measured values, the considered second device (25) being able to detect a physiological disorder of the patient (P) on the basis of at least one of the measured values and the communication between the centralizing device (20) and the at least one second device (25) occurring at radiofrequency.

2. Implantable system (10) comprising:
- a first device (20), called the centralizing device, able to be attached in an attachment position to a wall of the stomach (30) of a patient (P), the centralizing device (20) being accommodated in the stomach (30) when the centralizing device (20) is in the attachment position,
- the implantable system (10) comprising two second devices (25),
the implantable system (10) being **characterized in that** the centralizing device (20) comprises a controller (45), an electrical power supply (55) and a transceiver (60) able to allow communication between the centralizing device (20) and each of the second devices (25) when each of the second devices (25) is in a working position, at least one of the second devices (25) being located outside the body of the patient (P) when this at least one of the second devices (25) is in the working position, wherein:
- one of the second devices, also called the measuring device (25), comprises at least one sensor (117) able to measure a value of a physiological parameter of the patient (P), the transceiver (60) being configured to receive, from the measuring device (25) comprising a sensor (117), the measured values, and to transmit the measured values to the other second device, called the displaying device (25), the latter being able to detect a physiological disorder of the patient (P) on the basis of at least one of the values received from the transceiver (60), the communication between the centralizing device (20) and each of the second devices (25) occurring at radiofrequency.

3. Implantable system (10) according to either one of Claims 1 and 2, wherein the controller (45) comprises a memory (75) able to store the measured values for a time longer than or equal to one day.

4. Implantable system (10) according to any one of Claims 1 to 3, wherein the physiological disorder is a heart disease.

5. Implantable system (10) according to any one of Claims 1 to 4, wherein the sensor (117) is an ultrasound transceiver.

6. Implantable system (10) according to either one of Claims 3 and 4 in combination with Claim 1, wherein the sensor (117) is an accelerometer able to measure values of an acceleration of the centralizing device (20) and the second device (25) is configured to compute a physiological parameter of the heart of the patient (P) on the basis of the measured acceleration values.

7. Implantable system (10) according to either one of Claims 3 and 4 in combination with Claim 2, wherein the sensor (117) is an accelerometer able to measure values of an acceleration of the centralizing device (20) and the measuring device (25) is configured to compute a physiological parameter of the heart of the patient (P) on the basis of the measured acceleration values.

8. Implantable system (10) according to any one of Claims 1 to 4, wherein the sensor (117) is able to measure a difference in electrical potential, an acceleration, a noise, an orientation, a pH or a temperature.

9. Implantable system (10) according to any one of Claims 1 to 4, wherein the centralizing device (20) comprises a catheter able to deliver a bodily fluid (F) of the patient (P) to the sensor (117), the sensor (117) being able to measure a content of a biological marker in the bodily fluid (F).

10. Implantable system (10) according to any one of Claims 1 to 4, wherein the sensor (117) comprises at least one light source and at least one detector of luminous radiation, the light source being configured to illuminate at least one segment of an organ of the patient (P) with luminous radiation, the detector being configured to measure a value of a coefficient of reflection of the luminous radiation, the controller (45) being configured to compute a degree of oxygenation of the blood flowing through the organ illuminated by the light source, on the basis of the coefficient of reflection.

11. Implantable system (10) according to any one of Claims 3 to 10 in combination with Claim 1, comprising a plurality of sensors (117), wherein the second device (25) is able to detect the physiological disorder on the basis of values of at least two sensors (117).

12. Implantable system (10) according to any one of Claims 3 to 10 in combination with Claim 2, comprising a plurality of sensors (117), wherein the measuring device (25) is able to detect the physiological disorder on the basis of values of at least two sensors (117).

13. Implantable system (10) according to any one of Claims 1 to 12, wherein the electrical power supply (55) comprises a removable store (90) of electrical energy and a connector (85) able to accommodate the store (90) of electrical energy, the store (90) of electrical energy being able to electrically power the controller (45) when the store (90) of electrical energy is electrically connected to the connector (85) in a connected position and preferably being configured to be swallowed by the patient (P) and to move spontaneously to the connected position from a disconnected position in which the store (90) of electrical energy is accommodated in the stomach (30) of the patient (P) and is disconnected from the connector (85).

14. Implantable system (10) according to any one of Claims 1 to 13, wherein the electrical power supply (55) comprises a generator of electrical energy able to generate an electrical current via reaction of at least one chemical species present in the body of the patient (P), in particular glucose.

15. Implantable system (10) according to any one of Claims 1 to 14, wherein the electrical power supply (55) comprises a generator of electrical energy able to generate an electrical current via conversion of mechanical energy into electrical energy.
